(19) Europäisches Patentamt
European Patent Office
Office européen des brevets

(11) **EP 2 614 056 B1**

(12) **EUROPÄISCHE PATENTSCHRIFT**

(45) Veröffentlichungstag und Bekanntmachung des Hinweises auf die Patenterteilung:
**23.07.2014 Patentblatt 2014/30**

(21) Anmeldenummer: **11749864.2**

(22) Anmeldetag: **05.09.2011**

(51) Int Cl.:
*C07D 313/04* (2006.01)    *C07C 29/149* (2006.01)

(86) Internationale Anmeldenummer:
**PCT/EP2011/065276**

(87) Internationale Veröffentlichungsnummer:
**WO 2012/032002 (15.03.2012 Gazette 2012/11)**

(54) **VERFAHREN ZUR HERSTELLUNG VON EPSILON-CAPROLACTON UND 1,6-HEXANDIOL**

METHODS FOR THE PRODUCTION OF EPSILON-CAPROLACTONE AND 1,6-HEXANEDIOL

PROCÉDÉS DE PRODUCTION D'EPSILON-CAPROLACTONE ET DE 1,6-HEXANEDIOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.09.2010 EP 10175795**

(43) Veröffentlichungstag der Anmeldung:
**17.07.2013 Patentblatt 2013/29**

(73) Patentinhaber: **BASF SE**
**67056 Ludwigshafen (DE)**

(72) Erfinder:
• **ABILLARD, Olivier**
  **68159 Mannheim (DE)**
• **BREUNINGER, Daniel**
  **67240 Bobenheim-Roxheim (DE)**
• **KLEINMANN, Eva**
  **68163 Mannheim (DE)**
• **PINKOS, Rolf**
  **67098 Bad Dürkheim (DE)**

(56) Entgegenhaltungen:
WO-A1-97/31883      WO-A1-99/25872
WO-A1-2008/152001

## Beschreibung

Die vorliegende Erfindung betrifft ein Verfahren zur Herstellung von ε-Caprolacton mit mindestens 99 %iger Reinheit, und zur Herstellung 1,6-Hexandiol aus einer Dicarbonsäurelösung (DCL), umfassend die Schritte (a) Veresterung der DCL mit Alkohol, (b) partielle katalytische Hydrierung der Ester, (c) destillative Abtrennung des 1,6-Hexandiols, (d) Cyclisierung des in der Sumpffraktion enthaltenen 6-Hydroxycapronsäureesters, und (e) destillative Reinigung des ε-Caprolacton aus dem Destillat der Cyclisierung, wobei das Verfahren so durchgeführt wird, dass die Cyclisierung in Gegenwart eines bezogen auf ε-Caprolacton höher siedenden Alkohols stattfindet, und wobei die Cyclisierung in der Flüssigphase in einer Apparatur mit Kolonne mit mehr als einer theoretischen Trennstufe durchgeführt wird. Insbesondere betrifft die Erfindung ein Verfahren bei dem der bezogen auf ε-Caprolacton höher siedenden Alkohol bereits zur Veresterung der DCL (Schritt (a)) eingesetzt wird, sowie ein Verfahren bei dem die Bedingungen der Hydrierung (Schritt (b) und/oder der anschließenden Destillation (Schritt (c)) so gewählt sind, dass im Verfahren entstehendes 1,6-Hexandiol ursprünglich zur Veresterung eingesetzte leichter siedende Alkohole durch Umesterung verdrängt. Die Erfindung betrifft ferner Ausführungsformen dieses Verfahrens, bei der das 1,6-Hexandiol von Schritt (c) destillativ weiter gereinigt werden.

ε-Caprolacton bzw. die daraus durch Polyaddition hergestellten Polycaprolactone dienen zur Herstellung von Polyurethanen. 1,6-Hexandiol stellt einen Monomerbaustein dar, der überwiegend auf dem Polyester- und Polyurethansektor eingesetzt wird.

Der Verbund mit Produktionsanlagen zur Herstellung von Cyclohexanol und Cyclohexanon und die Nutzung des Abfallprodukts Dicarbonsäurelösung (DCL) als Ausgangsprodukt für die Herstellung von ε-Caprolacton und 1,6-Hexandiol führt gegenüber reiner 6-Hydroxycapronsäure bzw. reiner Adipinsäure zu günstigen Einsatzstoffkosten. Sie stellt außerdem eine umweltfreundliche Nutzung eines Abfallprodukts dar.

Die wässrige Dicarbonsäurelösung (DCL), die bei der Oxidation von Cyclohexan zu Cyclohexanol und Cyclohexanon (vgl. Ullmann's Encyclopedia of Industrial Chemistry, 5. Ed., 1987, Vol. A8, S. 219) als Nebenprodukte entsteht, enthält (berechnet wasserfrei in Gew.-%) im Allgemeinen zwischen 10 und 40 % Adipinsäure, zwischen 10 und 60 % 6-Hydroxycapronsäure, zwischen 1 und 10 % Glutarsäure, zwischen 1 und 10 % 5-Hydroxyvaleriansäure, zwischen 0,5 und 5 % 5-Formylvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im Allgemeinen 5 % nicht übersteigen. Beispielsweise seien Essigsäure, Propionsäure, Buttersäure, Valeriansäure, Capronsäure, Oxalsäure, Malonsäure, Dihydromuconsäure, Bernsteinsäure, 4-Hydroxy-buttersäure, γ-Butyrolacton und ε-Caprolacton als Mono- und dicarbonsäuren, Ester, Oxo- und Oxa-Verbindungen genannt. Wünschenswert ist es, DCL für die Gewinnung von 1,6-Hexandiol und reinem ε-Caprolacton, ausgehend von der in der DCL enthaltenen 6-Hydroxycapronsäure und Adipinsäure, zu verwenden.

Der bisher genannte Stand der Technik zeigt, dass sich Adipinsäurediester, hergestellt aus DCL und Monoalkoholen mit hohen Ausbeuten von etwa 96 bis 99 % zu 1,6-Hexandiol hydrieren lassen. Für die Hydrierung wurden dabei Katalysatoren auf der Basis von Kupfer und Zinkoxid, Kupfer und Aluminiumoxid oder Kupfer, Aluminiumoxid und Zinkoxid verwendet.

WO 99/25672 A1 beschreibt ein Verfahren zur Herstellung von 6-Hydroxycapronsäureester aus DCL, wobei die DCL zunächst verestert und dann katalytisch partiell hydriert wird. Vom Hydrieraustrag wird 1,6-Hexandiol destillativ entfernt und anschließend 6-Hydroxycapronsäureester abgetrennt.

Die Herstellung von Caprolacton aus DCL ist z.B. in DE 1 618 143 bereits beschrieben worden. Dabei wird entwässerte DCL mit Phosphorsäure thermisch umgesetzt und ein Gemisch aus Dicarbonsäuren, Caprolacton sowie eine Vielzahl anderer Komponenten fraktioniert. Der Sumpf fällt dabei z.T. fest und schwerlöslich an. Das Caprolacton hat aber auch nach weiterer destillativer Aufarbeitung nur eine 98 %ige Reinheit.

Ferner ist vielfach beschrieben, 6-Hydroxycapronsäure oder deren Ester zu Caprolacton umzusetzen (z.B. DE 2 013 525, EP-A 349 861 und darin zitierte Literatur).

Im Ausführungsbeispiel von DE 196 07 954 (auch als WO 97/31883 A1 veröffentlicht) ist beschrieben, entwässerte DCL mit Methanol zu verestern und das Estergemisch nach Abtrennung von Leichtsiedern in eine Adipinsäuredimethylester-Fraktion und eine Hydroxycapronsäuremethylester-Fraktion aufzutrennen. Die Adipinsäuredimethylester-Fraktion wurde demnach in Gegenwart von Kupfer, Zinkoxid und Aluminiumoxid enthaltenden Katalysatoren zu 1,6-Hexandiol und Methanol hydriert, wobei der Esterumsatz bei 220 °C und 220 bar 99,5 %, die 1,6-Hexandiol-Selektivität > 99 % betrugen. Die Hydroxycapronsäuremethylester-Fraktion wurde katalytisch zu Caprolacton cyclisiert, wobei die Reinheit nach abschließender Destillation 99,9 % betrug. Damit, bei der Wahl von Methanol oder anderen leicht siedenden Alkoholen für die Veresterung der DCL, die Cyclisierung des 6-Hydroxycapronsäureesters zum Caprolacton in befriedigender Ausbeute erfolgen kann, muss vor der Cyclisierung der Adipinsäureester vollständig von der 6-Hydroxycapronsäureester-Fraktion abgetrennt werden. Um dies sicherzustellen ist einerseits ein hoher Trennaufwand erforderlich und andererseits eine Fahrweise notwendig, bei der ein Teil des 6-Hydroxycapronsäureesters zusammen mit dem Adipinsäureester abgetrennt wird und somit für die Cyclisierung verloren geht. Die Verwendung von höher siedenden Alkoholen für die Veresterung der DCL würde in diesem Verfahren die Trennung der Adipinsäureester-Fraktion und der 6-Hydroxycapronsäureester-Fraktion erschweren. Nachteilig an diesem Verfahren mit der Verwendung von Methanol oder anderen leicht siedenden Alkoholen für die Veresterung der DCL ist auch, dass die Veresterung zur Vermeidung von sehr hohen Drücken bei vergleichsweise niedrigen Temperaturen (< 200°C) durchgeführt werden muss, weshalb üblicherweise ein Katalysator, in der Regel Schwefelsäure, zu verwenden ist, um eine effektive Veresterung zu gewährleisten. Die Schwefelsäure muss in diesen Fällen nach der Veresterung aber wieder aufwendig entfernt werden. Weiterhin gelingt die Abtrennung von Reaktionswasser bei Verwendung von leicht siedenden Alkoholen nur mit sehr großem Aufwand, so dass dies in der Praxis nicht durchgeführt wird. Die Anwesenheit von Wasser in der Veresterung verursacht dann schlechtere Umsätze, so dass noch kleinere Säuremengen im Veresterungsaustrag enthalten sind. Diese führen in späteren Prozessstufen (besonders in Destillationen) zur Bildung von besonders Polyhydroxycapronsäureestern in den Sumpffraktionen. Diese müssen entweder aufwendig aufgearbeitet und zurückgeführt werden oder sind für die weitere Verarbeitung zum Caprolacton oder Hexandiol verloren.

Weiter beschreibt DE 196 07 954 eine Verfahrensvariante ("Variante E") insbesondere für die Herstellung kleiner Mengen von Caprolacton mit minimalem technischen Aufwand. Demnach ist für die Veresterung der DCL ein Alkohol zu verwenden, der höher als Caprolacton siedet, und die Umsetzung wird ohne Isolierung der Adipinsäureester-Fraktion ansatzweise in einer Eintopfreaktion in Gegenwart eines Veresterungskatalysators durchgeführt. Das Caprolacton kann dann als Destillat gewonnen werden, während der hoch siedenden Adipinsäureester im Sumpf verbleibt. Nachteilig sind hierbei die diskontinuierliche Verfahrensführung und eine nur mäßige Reinheit des so erhältlichen Caprolactons (ca. 98 %).

WO 2008/152001 A1 wiederum beschreibt ein Verfahren zur Cyclisierung von 6-Hydroxycapronsäureester aus einem Adipinsäureester haltigen Gemisch in der Gasphase.

Als der Erfindung zugrunde liegende Aufgabe kann daher erachten werden - ausgehend von DCL - 1,6-Hexandiol und sehr reines ε-Caprolacton herzustellen und dabei die Nachteile des Standes der Technik, d.h. entweder hohe Kosten der Herstellung oder unzureichende Reinheit und Ausbeute der Produkte, zu vermeiden. Diese Aufgabe wird gelöst durch die im Folgenden beschriebenen und durch die beanspruchten Ausführungsformen.

Entsprechend betrifft die vorliegende Erfindung ein Verfahren zur Herstellung 1,6-Hexandiol und sehr reinem ε-Caprolacton aus einer Dicarbonsäurelösung (DCL) umfassend die Schritte

(a) Veresterung der DCL mit Alkoholen,
(b) partielle katalytische Hydrierung der Ester,
(c) destillative Abtrennung des 1,6-Hexandiols als Kopfprodukt,
(d) Cyclisierung des in der Sumpffraktion enthaltenen 6-Hydroxycapronsäureesters zum ε-Caprolacton in Gegenwart eines Alkohols mit einem Siedepunkt größer dem des ε-Caprolactons, und
(e) Reinigung des ε-Caprolactons aus dem Destillat von Schritt (d) mittels Destillation, wobei die Cyclisierung in Schritt (d) in der Flüssigphase in einer Apparatur mit Kolonne mit mehr als einer theoretischen Trennstufe durchgeführt wird.

Insbesondere betrifft die vorliegende Erfindung ein Verfahren zur Herstellung 1,6-Hexandiol und sehr reinem ε-Caprolacton aus einer Dicarbonsäurelösung (DCL) umfassend die Schritte

(a) Veresterung der DCL mit Alkoholen,
(b) partielle katalytische Hydrierung der Ester,
(c) destillative Abtrennung des 1,6-Hexandiols als Kopfprodukt,
(d) Cyclisierung des in der Sumpffraktion enthaltenen 6-Hydroxycapronsäureesters zum ε-Caprolacton in Gegenwart eines Alkohols mit einem Siedepunkt größer dem des ε-Caprolactons, und
(e) Reinigung des ε-Caprolactons aus dem Destillat von Schritt (d) mittels Destillation, wobei für die Veresterung in Schritt (a) bereits Alkohole mit einem Siedepunkt größer dem des ε-Caprolactons verwendet werden, und
wobei die Cyclisierung in Schritt (d) in der Flüssigphase in einer Apparatur mit Kolonne mit mehr als einer theoretischen Trennstufe durchgeführt wird.

Weiter betrifft die vorliegende Erfindung ein Verfahren zur Herstellung 1,6-Hexandiol und sehr reinem ε-Caprolacton aus einer Dicarbonsäurelösung (DCL) umfassend die Schritte

(a) Veresterung der DCL mit Alkoholen,
(b) partielle katalytische Hydrierung der Ester,
(c) destillative Abtrennung des 1,6-Hexandiols als Kopfprodukt,
(d) Cyclisierung des in der Sumpffraktion enthaltenen 6-Hydroxycapronsäureesters zum ε-Caprolacton in Gegenwart eines Alkohols mit einem Siedepunkt größer dem des ε-Caprolactons, und
(e) Reinigung des ε-Caprolactons aus dem Destillat von Schritt (d) mittels Destillation, wobei für die Hydrierung in Schritt (b) und/oder die Destillation in Schritt (c) unter Bedingungen durchgeführt werden, die eine Verdrängung von gegebenenfalls leichter siedenden Veresterungsalkoholen aus Schritt (a) durch das bei der Hydrierung entstehende 1,6-Hexandiol im Sinne einer Umesterung ermöglichen, und

wobei die Cyclisierung in Schritt (d) in der Flüssigphase in einer Apparatur mit Kolonne mit mehr als einer theoretischen Trennstufe durchgeführt wird.

Solche Bedingungen sind beispielsweise die Verwendung von Hydrierkatalysator mit sauren oder basischen Zentren für die Hydrierung in Schritt (b), die Gegenwart von in geringe Mengen von Säuren oder Basen (Säure- bzw. Basenzahl im Zulauf zur Destillationsstufe (c) von mindestens 0,01) bei der Destillation von Schritt (c), und/oder die Gegenwart von Umesterungskatalysatoren - z.B. Natriummethylat - (in Mengen von mindestens 1 ppm) bei der Hydrierung von Schritt (b) und/oder der Destillation von Schritt (c).

Das erfindungsgemäße Verfahren zur Herstellung von 1,6-Hexandiol und sehr reinem ε-Caprolacton verwendet als Ausgangsmaterial Dicarbonsäurelösungen (DCL). Solche DCL entstehen in Form wässriger Lösungen als Nebenprodukte der Oxidation von Cyclohexan zu Cyclohexanol und Cyclohexanon (vgl. Ullmann's Encyclopedia of Industrial Chemistry, 5. Ed., 1987, Vol. A8, S. 219). Sie enthalten (berechnet wasserfrei in Gew.-%) im Allgemeinen zwischen 10 und 40 % Adipinsäure, zwischen 10 und 60 % 6-Hydroxycapronsäure, zwischen 1 und 10 % Glutarsäure, zwischen 1 und 10 % 5-Hydroxyvaleriansäure, zwischen 0,5 und 5 % 5-Formylvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im Allgemeinen 5 % nicht übersteigen. Beispielsweise seien Essigsäure, Propionsäure, Buttersäure, Valeriansäure, Capronsäure, O-xalsäure, Malonsäure, Dihydromuconsäure, Bernsteinsäure, 4-Hydroxybuttersäure, γ-Butyrolacton und Caprolacton als Mono- und dicarbonsäuren, Ester, Oxo- und Oxa-Verbindungen genannt. Die DCL ist im Allgemeinen eine wässrige Lösung mit einem Wasseranteil von 20 bis 80 Gew.-%.

Aldehyde wie 5-Formylvaleriansäure und Ketone wie 1,4-Cyclohexandion und 4-Hydroxycyclohexanon können bei der Veresterung mit Diolen (Schritt (a)) Acetale und Ketale bilden. Hierdurch kann 5-Formylvaleriansäure durch Folgereaktionen der Acetale für die Herstellung von ε-Caprolacton und 1,6-Hexandiol verloren gehen. Durch die Acetal- oder Ketalbildung kann der jeweils gebundene Alkohol ganz oder teilweise verloren gehen.

Je nach Zusammensetzung der DCL kann es daher vorteilhaft sein, die enthaltenen Aldehyde und Ketone vor dem Veresterungsschritt (a) katalytisch zu Alkoholen zu hydrieren. Wurde die Cyclohexan-Oxidation in Abwesenheit eines Deperoxidations-Katalysators, wie beispielsweise Cobaltnaphthenat, durchgeführt, so enthält die DCL wie in DE-A 1 951 250 und EP-A 847 979 beschrieben 6-Hydroperoxycapronsäure. Wurde in Anwesenheit eines Deperoxidations-Katalysators oxidiert, so ist 6-Hydroperoxycapronsäure nur in geringen Mengen enthalten. Wurde die Cyclohexan-Oxidation ohne Katalysator durchgeführt, so kann die entstandene 6-Hydroperoxycapronsäure ebenso wie 5-Formylvaleriansäure zu 6-Hydroxycapronsäure hydriert werden. Diese optionale Hydrierung findet dann vor dem Schritt (a) des erfindungsgemäßen Verfahrens statt.

Da bei der Hydrierung, die gegebenenfalls vor dem Schritt (a) des erfindungsgemäßen Verfahrens stattfindet, im einen Fall Hydroperoxy-, im anderen Fall eine Aldehydgruppe hydriert werden muss, unterscheiden sich die optimalen Hydrierbedingungen beider Verbindungen. Da die Hydroperoxycapronsäure auch rein thermisch, aber weniger selektiv als bei einer Hydrierung, in 6-Hydroxycapronsäure übergehen kann, wird sie gemäß DE-A 1 951 250 in Gegenwart von Palladium, Rhodium- oder Platin-Katalysatoren bei 15 bis 130°C, vorzugsweise 50 bis 100°C, also bei moderaten Temperaturen hydriert. Keto- und Aldehydgruppen werden unter den Bedingungen der 6-Hydroperoxycapronsäure-Hydrierung in DE-A 1 951 250 nicht hydriert. Hierfür sind höhere Temperaturen und Drücke notwendig.

Die Hydrierung, die gegebenenfalls vor Schritt (a) des erfindungsgemäßen Verfahrens durchgeführt wird, erfolgt bei 10 bis 200°C, bevorzugt 30 bis 180°C, besonders bevorzugt 50 bis 170°C. Der Wasserstoff-Partialdruck beträgt dabei 1 bis 100 bar, bevorzugt 10 bis 80 bar, besonders bevorzugt 30 bis 60 bar.

Für die katalytische Hydrierung, die gegebenenfalls vor Schritt (a) des erfindungsgemäßen Verfahrens durchgeführt wird, dienen Katalysatoren, die mindestens ein Metall der 7. bis 12. Gruppe des Periodensystems, beispielsweise Ruthenium, Palladium, Rhodium, Nickel, Cobalt, Eisen, Rhenium, Platin, Iridium, Kupfer, Osmium und Zink enthalten. Gut geeignet sind weiterhin sogenannte Vollkatalysatoren, die keine Träger enthalten und aus Metallen, Metalloxiden oder deren Gemischen bestehen. Bevorzugt sind dabei Eisen- und insbesondere Cobalt-Vollkatalysatoren.

Für die Veresterung gemäß Schritt (a) des erfindungsgemäßen Verfahrens der in der DCL enthaltenen Carbonsäuren sind Alkohole mit 1 bis 30 C-Atomen verwendbar, bevorzugt sind solche, die im Druckbereich von 10 bis 1500 mbar höher sieden als ε-Caprolacton (beispielsweise 96-97°C bei 20 mbar oder 235°C bei 1013 mbar). Dabei können Monoalkohole sowie Diole und andere mehrwertige Alkohole verwendet werden. Ebenso können Gemische solcher Alkohole oder Zusammensetzungen, die solche Alkohole enthalten (bevorzugt zu einem Anteil von mindestens 30 Gew.-%, besonders bevorzugt zu einem Anteil von mindestens 50 Gew.-%), verwendet werden. Beispiele für derartige Alkohole sind Glycerin, Trimethylolpropan, 1,6-Hexandiol, 1,8-Octandiol, 1,10-Decandiol, 1,12-Dodecandiol, 1-Tridecanol, 1-Tetradecanol, 1-Pentadecanol, 1-Hexadecanol. 1-Octadecanol, 1-Eicosanol, Butylethylpropandiol, Neopentylglycol, Pentaerythrit, Triethylenglycol, Tetraethylenglycol, Bis(6-hydroxyhexyl)ether. Besonders geeignet sind mehrwertige Alkohole, insbesondere Diole, insbesondere α,ω-Diole mit sechs bis zwölf Kohlenstoffatomen. Besonders bevorzugt ist 1,6-Hexandiol, da dieser Veresterungsalkohol einem Zielprodukt des erfindungsgemäßen Verfahrens entspricht.

Die Verwendung von Alkoholen, die höher sieden als ε-Caprolacton, für die erfindungsgemäße Veresterung der DCL ist vorteilhaft, um bei dem Cyclisierungsschritt (d) des erfindungsgemäßen Verfahrens in Gegenwart von Adipinsäure bzw. Adipinsäureester eine befriedigende Umsetzung des 6-Hydroxycapronsäureesters zu erzielen. Sofern als Veresterungsalkohol ein niedrig siedender Alkohol verwendet wurde, beispielsweise Methanol, so ist das erfindungsgemäße Verfahren ebenfalls anwendbar, vorausgesetzt Hydrierungsschritt (b) und/oder Destillationsschritt (c) werden so durchgeführt, dass bei der Hydrierung gebildetes 1,6-Hexandiol als gegenüber Caprolacton höher siedender Alkohol den ursprünglichen Veresterungsalkohol als Leichtsieder aus seinen Estern verdrängt und damit im Cyclisierungsschritt (d) gegenwärtig ist, so dass die Cyclisierung in Gegenwart von Adipinsäure-Äquivalenten effektiv erfolgen kann.

Damit während der Hydrierung und anschließenden Destillation das gebildete Hexandiol den niedrig siedenden Alkohol aus seinen Estern verdrängen kann, müssen gewisse Voraussetzungen erfüllt sein. Der Hydrierkatalysator sollte über saure oder basische Zentren verfügen, entweder Lewis- und/oder Brönstedt-Zentren. Sollte der Hydrierkatalystor über keine oder nur zuzureichend umesternde katalytische Zentren verfügen, so kann dem Hydrierfeed ein Umesterungskatalysator zugesetzt werden, beispielsweise Natriummethylat. Dieser Zusatz wird in Mengen bezogen auf den Zulauf von 1 bis 1000 ppm, bevorzugt 5 bis 500 ppm, besonders bevorzugt 10 bis 300 ppm zugesetzt. Damit bei der Destillation Hexandiol niedrig siedende Alkohole aus ihren Estern freisetzen kann, sollten in geringe Mengen Säuren oder Basen zugegen sein. Dies kann z.B. bereits eine Carbonsäure wie Adipinsäure oder Adipinsäurehalbester oder Hydroxycapronsäure sein, deren Gehalt eine Säurezahl von 0,01 bis 5 im Zulauf zur Stufe (c) verursacht. Ferner sind natürlich auch Zusätze wie Natriummethylat oder Titanate wie Tetra-n-butyltitant möglich, in Mengen von 1 bis 5000 ppm, bevorzugt 5 bis 3000 ppm, besonders bevorzugt 10 bis 2000 ppm.

In einer Ausführungsform des erfindungsgemäßen Verfahrens wird, anstelle von reinem 1,6-Hexandiol, ein Teil des Hydrieraustrags von Schritt (b) für die Veresterung der DCL gemäß Schritt (a) verwendet. Der Hydrieraustrag ist eine alkoholische Zusammensetzung, die im Allgemeinen 30 bis 90 Gew.-% 1,6-Hexandiol, 1 bis 10 Gew.-% 1,5-Pentandiol, 1,4-Butandiol, 1,2-Cyclohexandiol und 1,4-Cyclohexandiol (jeweils weniger als 5 Gew.-%), weiterhin bis 5 Gew.-% Monoalkohole wie z.B. n-Butanol, n-Pentanol und n-Hexanol und 1 bis 50 Gew.-% oligomere oder polymere Hochsieder gegenüber 1,6-Hexandiol enthält.

In einer weiteren Ausführungsform des erfindungsgemäßen Verfahrens wird ein Teil des Kopfprodukts aus dem Destillationsschritt (c) als alkoholische Zusammensetzung für die Veresterung der DCL in Schritt (a) verwendet.

Die Verwendung von Diolen, insbesondere von α,ω-Diolen als Veresterungsalkohole stellt einen Vorteil gegenüber Monoalkoholen dar. Die Verluste an Veresterungsalkohol verringern sich und die Aufarbeitung des Hydrieraustrags wird vereinfacht.

Der DCL wird zur Veresterung in Schritt (a) des erfindungsgemäßen Verfahrens, bevorzugt ein Alkohol, der höher siedet als ε-Caprolacton, insbesondere ein entsprechendes α,ω-Diol wie 1,6-Hexandiol oder ein Gemisch solcher Alkohole oder eine solche Alkohole enthaltene Zusammensetzung (bevorzugt mit einem Anteil solcher Alkohole von mindestens 30 Gew.-%, besonders bevorzugt mit einem Anteil von mindestens 50 Gew.-%) hinzugefügt. Als eine solche Zusammensetzung kann eine Teilmenge des Hydrieraustrags oder eine Teilmenge des Kopfprodukts der destillativer Abtrennung (Schritt (c)) dienen. Das Massenverhältnis von DCL zu Alkohol beträgt bei der Verwendung von Diolen dabei bevorzugt 1 zu 0,2 bis 1 zu 0,8, insbesondere 1 zu 0,3 bis 1 zu 0,7, und besonders bevorzugt 1 zu 0,4 bis 1 zu 0,6. Bei Verwendung von Monoalkoholen ist die Alkoholmenge um den Faktor zwei zu erhöhen, bei Polyolen ist die Alkoholmenge entsprechend zu reduzieren, z.B. bei Triolen um Faktor 1,5, bei Tetraolen um Faktor 2, Pentaolen Faktor 2,5, Hexaolen Faktor 3 usw. Bei der Verwendung von Alkohol enthaltenden Zusammensetzungen für die Veresterung (z.B. der Hydrieraustrag von Schritt (b)) ist eine entsprechende - auf deren Alkoholanteil bezogen - erhöhte Menge der Zusammensetzung zu wählen.

Die Abtrennung des Wassers von der wässrigen DCL und Veresterung gemäß Schritt (a) des erfindungsgemäßen Verfahrens werden bevorzugt in einem Verfahrensschritt durchgeführt. Hierzu können Rührreaktoren, Strömungsrohre und/oder Kolonnen verwendet werden. Bevorzugt erfolgen Wasserabtrennung und Veresterung in mindestens einem Reaktor mit aufgesetzter Destillationskolonne. Um einen vollständigen Umsatz bei der Veresterung der Carbonsäuren und eine vollständige Wasserabtrennung zu erreichen, arbeitet man mit 2 bis 6, bevorzugt mit 3 bis 5 hintereinander geschalteten Reaktoren mit aufgesetzter Kolonne.

Die Veresterungsreaktion der DCL gemäß Schritt (a) des erfindungsgemäßen Verfahrens kann ohne Zusatz eines Katalysators ablaufen. Es kann aber auch zur Erhöhung der Reaktionsgeschwindigkeit ein Katalysator für die Veresterung zugesetzt werden. Dabei kann es sich um einen homogenen, gelösten oder um einen heterogenen Katalysator handeln.

Als homogene Katalysatoren für die Veresterung seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z.B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäure beträgt in der Regel 0,0001 bis 0,5, bevorzugt 0,001 bis 0,3.

Als heterogene Katalysatoren sind saure oder supersaure Materialien, z.B. saure und supersaure Metalloxide wie $SiO_2$, $Al_2O_3$, $SnO_2$, $ZrO_2$, Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Bevorzugt wird ohne Katalysator-Zusatz verestert.

Die Sumpftemperatur in den Reaktoren mit aufgesetzter Kolonne beträgt 200 bis 250°C. Die Veresterung erfolgt unter gleichzeitiger Abtrennung des in DCL enthaltenen Wassers sowie des Reaktionswassers. Die Veresterung und Wasserabtrennung kann bei Drücken von 0,1 bis 5 bar, bevorzugt 0,5 bis 3 bar, besonders bevorzugt bei 0,8 bis 1,3 bar durchgeführt werden. Die Verweilzeit, gerechnet über alle Rührkessel beträgt 0,5 bis 12 Stunden, bevorzugt 1 bis 11 Stunden, besonders bevorzugt 2 bis 10 Stunden. Die Veresterung kann kontinuierlich oder diskontinuierlich durchgeführt werden

Als Kopfprodukt der aufgesetzten Kolonnen wird das in der DCL enthaltene und das bei der Veresterung entstandene Wasser erhalten. Das Kopfprodukt kann weiterhin organische Nebenprodukte wie z.B. niedere Monocarbonsäuren, z.B. Ameisensäure, enthalten.

Als Sumpfprodukt des letzten Reaktors fällt ein Estergemisch (im Falle der Verwendung von Diolen, ein Gemisch aus Oligo- und Polyestern) an, das sich aus den in der DCL enthaltenen Carbonsäuren, den Cyclohexandiolen und den zugesetzten Alkoholen bildete. Im Sumpfprodukt sind außerdem nicht umgesetzte Alkohole enthalten. Dieses Gemisch wird für die anschließende katalytische Hydrierung in Schritt (b) des erfindungsgemäßen Verfahrens verwendet.

Die Vollständigkeit des Umsatzes der in dem Carbonsäuregemisch vorhandenen freien Carboxylgruppen wird mit der nach der Veresterung gemessenen Säurezahl (mg KOH/g) festgestellt. Sie beträgt abzüglich der gegebenenfalls zugesetzten Säure als Katalysator 1 bis 20, bevorzugt 2 bis 15, besonders bevorzugt 5 bis 10 mg KOH/g.

Wurde zur Veresterung eine gelöste Säure als Katalysator eingesetzt, wird das Estergemisch zweckmäßiger Weise mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet. Die zugesetzte Säure kann auch durch überleiten des Estergemisches über einen schwach basischen Ionentauscher entfernt werden.

Die Hydrierung des Estergemischs in Schritt (b) des erfindungsgemäßen Verfahrens erfolgt katalytisch in der Flüssigphase in Gegenwart von fest angeordneten oder suspendierten, bevorzugt fest angeordneten Katalysatoren. Gearbeitet wird bei Temperaturen zwischen 100 und 350°C, bevorzugt 120 und 300°C, besonders bevorzugt 140 und 280°C und Drücken von 30 bis 350 bar, bevorzugt 40 bis 320 bar, besonders bevorzugt 50 bis 300 bar. Die KatalysatorBelastung beträgt 0,2 bis 1,5 kg Ester pro kg Katalysator und Stunde.

Die Hydrierung in Schritt (b) erfolgt erfindungsgemäß partiell, also mindestens zu einem Teil, nicht aber vollständig.

Der Umsatz der Hydrierung in Schritt (b) des erfindungsgemäßen Verfahrens wird bewusst beschränkt, um für die Cyclisierung zum ε-Caprolacton (Schritt (d)) einen höheren Anteil an 6-Hydroxycapronsäureestern sicherzustellen. Bevorzug wird eine partielle Hydrierung mit einem Hydrierumsatz im Bereich von 40 bis 97 %, besonders bevorzugt von 50 bis 95 % eingestellt. Dies kann beispielsweise durch Änderung der Hydriertemperatur, Erhöhung der Belastung, Erniedrigung des Drucks, oder prinzipiell auch durch Umfahren des Nachreaktors bewirkt werden.

Der Hydrierumsatz ist definiert als Verhältnis aus der Verringerung der Esterzahl (EZ) im Reaktionsgemisch während der Hydrierung zu der Gesamt-Esterzahl im Reaktionsgemisch vor der Hydrierung:

$$H\% = 100 * (EZ_{R,V} - EZ_{R,N}) / EZ_{R,V}$$

$EZ_{R,V}$: Esterzahl der Reaktionsmischung vor der Hydrierung
$EZ_{R,N}$: Esterzahl der Reaktionsmischung nach der Hydrierung
$H\%$: Hydrierumsatz in Prozent

Die Esterzahl (EZ) wird bestimmt aus der Differenz der Verseifungszahl (VZ) und der Säurezahl (SZ) der Mischung:

$$EZ_R = VZ_R - SZ_R$$

$EZ_R$: Esterzahl der jeweiligen Reaktionsmischung
$VZ_R$: Verseifungszahl der jeweiligen Reaktionsmischung
$SZ_R$: Säurezahl der jeweiligen Reaktionsmischung

Die Verseifungszahl (VZ) wird nach DIN 53401 und die Säurezahl (SZ) nach DIN 54302 (neue Version DIN EN ISO 2114) bestimmt.

Alternativ kann der Hydrierumsatz auch anhand anderer Methoden ermittelt werden, etwa aus dem Verbrauch von Wasserstoff im Reaktionsgemisch.

Der Hydrierumsatz bei Schritt (b) des erfindungsgemäßen Verfahrens kann für die jeweiligen Ausgangsbedingungen (Beschaffenheit der Anlage, des Katalysators, und der Ausgangssubstanzen) auf den gewünschten Wert eingestellt werden. Wird ein zu hoher Hydrierumsatz festgestellt, so kann dieser reduziert werden durch schrittweise Absenkung der Hydriertemperatur, des Reaktordrucks oder der zugefahrenen Wasserstoffmenge pro Zeiteinheit, oder durch Erhöhung der Zulaufmenge pro Zeiteinheit. Umgekehrt kann, wenn ein zu niedriger Hydrierumsatz festgestellt wird, dieser erhöht werden durch schrittweise Erhöhung der Hydriertemperatur, des Reaktordrucks, oder der zugefahrenen Wasserstoffmenge pro Zeiteinheit, oder durch Absenkung der Zulaufmenge pro Zeiteinheit. Dabei kann eine einzelne Änderung vorgenommen werden oder auch mehrere. Nach jeder Änderung muss nach ausreichender Zeit der neu eingestellte Hydrierumsatz bestimmt werden und gegebenenfalls müssen weitere Änderungen vorgenommen werden.

Die Hydrierung in Schritt (b) des erfindungsgemäßen Verfahrens kann grundsätzlich in nur einem Reaktor durchgeführt werden. Diese Fahrweise besitzt jedoch Nachteile: Esterhydrierungen sind stark exotherm und müssen dazu noch bei hohen Temperaturen durchgeführt werden. So beträgt die Hydriertemperatur nach US-A 3,524,892, wo die Hydrierung von aus DCL hergestellten Oligoestern in Gegenwart von durch Bariumoxid dotiertem Kupferchromit erfolgte, 260 bis 270°C. Für die sichere Wärmeabfuhr aus dem Reaktor muss ein hoher Aufwand getrieben werden. Daher wird die Hydrierung bevorzugt in mindestens zwei hintereinander geschalteten Reaktoren durchgeführt. Arbeitet man mit Festbettkatalysatoren, so kann man den Hydrierfeed in Sumpf- oder Rieselfahrweise über den Katalysator leiten. Beim Arbeiten in Sumpffahrweise wird Wasserstoffgas in den mit der flüssigen Reaktionsmischung gefluteten Reaktor eingeleitet, wobei der Wasserstoff das Katalysatorbett in aufsteigenden Gasperlen passiert. Beim Arbeiten in Rieselfahrweise lässt man das flüssige Estergemisch in dem Reaktor, der unter Wasserstoffdruck steht, über das in diesem angeordnete Katalysatorbett rieseln, wobei sich auf dem Katalysator ein dünner Flüssigkeitsfilm ausbildet. Nach einer bevorzugten Ausführungsform verwendet man mehrere Reaktoren, wobei im ersten Reaktor der überwiegende Teil der Ester hydriert wird. Der erste Reaktor wird bevorzugt mit Flüssigkeitskreislauf zur Wärmeabfuhr durch Wärmetauscher und der nachfolgende oder die nachfolgenden Reaktoren bevorzugt im geraden Durchgang, ohne Umlauf zur Vervollständigung des Umsatzes betrieben. Diese Fahrweise wird als Kreislauffahrweise bezeichnet.

Die Hydrierung in Schritt (b) des erfindungsgemäßen Verfahrens kann diskontinuierlich oder kontinuierlich erfolgen.

Die Hydrierung in Schritt (b) des erfindungsgemäßen Verfahrens wird im Allgemeinen mit dem bei der Veresterung anfallenden, überschüssige Alkohole enthaltenden Estergemisch ohne zusätzliches Lösungsmittel durchgeführt. Es kann aber auch vorteilhaft sein, in Gegenwart eines unter den Reaktionsbedingungen inerten Lösungsmittels zu arbeiten. Als Lösungsmittel kommen z.B. alle für die Veresterung verwendeten Alkohole, weiterhin Tetrahydrofuran, Dioxan und Monoalkohole mit 1 bis 6 Kohlenstoffatomen, wie z.B. Methanol, Ethanol, Propanole, n-Butanol, n-Hexanol oder Gemische der genannten Verbindungen in Frage. Die Lösungsmittel-Menge beträgt dabei 5 bis 50 Gew.-%, bevorzugt 10 bis 30 Gew.-%, bezogen auf das Estergemisch. Bevorzugt wird die Hydrierung ohne Lösungsmittel durchgeführt.

Es kann auch vorteilhaft sein, dem bei der Veresterung anfallenden Estergemisch eine Base zuzudosieren. Zum Einsatz kommen bevorzugt Lithium-, Natrium- und Kalium-Alkoholate, besonders bevorzugt Natriummethylat. Die Menge an Base beträgt dabei 20 bis 180 ppm, bevorzugt 30 bis 90 ppm, bezogen auf das Estergemisch. Bei einem Estergemisch mit Restsäurezahl > 1 mg KOH/g, werden die Restsäuren nur in unbedeutsamen Mengen neutralisiert. Die zugegebene Base dient dazu, die Bildung von Nebenprodukten, die sonst bei der Hydrierung entstehen könnten, wie z.B. Hexanol oder Etherverbindungen, zu unterdrücken.

Die Hydrierung in Schritt (b) des erfindungsgemäßen Verfahrens erfolgt in Gegenwart eines Katalysators. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind in H. Kropf, Houben-Weyl, Methoden der Organischen Chemie, Band IV/Ic, Georg Thieme Verlag Stuttgart, 1980, S. 45 bis 67, und Beispiele für heterogene Katalysatoren sind in Houben-Weyl, Methoden der Organischen Chemie, Band IV/Ic, S. 16 bis 26, beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. $Cr_2O_3$, $Al_2O_3$, $SiO_2$, $ZrO_2$, $TiO_2$, $ZnO_2$, BaO und MgO oder Mischungen daraus.

Bevorzugt sind Katalysatoren, wie sie in EP 0 552 463 beschrieben sind. Dies sind Katalysatoren, die in der oxidischen Form die Zusammensetzung $Cu_aAl_b Zr_cMn_dO_x$ besitzen, wobei $a > 0$, $b > 0$, $c \geq 0$, $d > 0$, $a > b/2$, $b > a/4$, $a > c$, $a > d$ gilt und x die zur Wahrung der Elektroneutralität pro Formeleinheit erforderliche Anzahl von Sauerstoffionen bezeichnet. Die Herstellung dieser Katalysatoren kann beispielsweise nach den Angaben der EP 0 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so dass man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfläche der Katalysatoren liegt zwischen 10 und 150 m$^2$/g.

In einer Ausführungsform werden für die Hydrierung in Schritt (b) des erfindungsgemäßen Verfahrens Katalysatoren verwendet, deren Formkörpervorläufer neben Kupferoxid, Aluminiumoxid und mindestens einem der Oxide des Lanthans, Wolframs, Molybdäns, Titans, Zirkoniums oder Eisens auch noch metallisches Kupfer, Kupferblättchen, pulverförmigen Zement, Graphit oder ein Gemisch enthält wie er bereits im Verfahren zur Hydrierung der Oligo- und Polyester der DCL mit Diolen beschrieben wurde. Der Katalysator und seine Herstellung sind in WO 2004/085356, WO 2006/005505 und WO 2007/006719 beschrieben.

In einer Ausführungsform wird die Hydrierung in Schritt (b) des erfindungsgemäßen Verfahrens durchgeführt mittels eines Katalysator-Formkörpers, dessen Vorläufer herstellbar ist, in dem

(i) ein oxidisches Material, umfassend Kupferoxid, Aluminiumoxid und mindestens eins der Oxide des Lanthans, Wolframs, Molybdäns, Titans, Zirkoniums oder Eisen bereitgestellt wird,

(ii) dem oxidischen Material pulverförmiges metallisches Kupfer, Kupferblättchen, pulverförmiger Zement, Graphit oder ein Gemisch davon zugegeben wird und

(iii) das aus (ii) resultierende Gemisch zu einem Formkörper verformt wird.

Insbesondere die Hydrierung von oligomeren Estern unterschiedlicher Zusammensetzung, wie sie bei der erfindungsgemäßen Veresterung einer DCL mit Diolen oder Diolgemischen entstehen, stellt hohe Anforderungen an den entsprechenden Katalysator, da dieser nicht vergiftet werden darf und trotzdem eine hohe Aktivität und Selektivtät zeigen muss. Die oben beschriebenen Katalysatoren, deren Formkörpervorläufer neben Kupferoxid, Aluminiumoxid und mindestens einem der Oxide des Lanthans, Wolframs, Molybdäns, Titans, Zirkoniums oder Eisens auch noch metallisches Kupfer, Kupferblättchen, pulverförmigen Zement, Graphit oder ein Gemisch enthält, erwies sich für diese Anwendung als besonders geeignet.

Die Formkörper werden vor dem Einsatz als Katalysator in an sich bekannter Weise durch Behandlung mit reduzierenden Medien wie z.B. Wasserstoff aktiviert. Das Aktivieren erfolgt entweder vorab in einem Reduktionsofen oder nach dem Einbau im Reaktor. Ist der Katalysatorvorläufer vorab im Reduktionsofen aktiviert worden, wird er in den Reaktor eingebaut und direkt unter Wasserstoffdruck mit der Hydrierlösung beschickt.

Der Hydrieraustrag von Schritt (b) hat im Allgemeinen eine Zusammensetzung von 30-90 Gew.-% 1,6-Hexandiol, 1-10 Gew.-% 1,5-Pentandiol, <5 % 1,4-Butandiol, <5 % 1,2-Cyclohexandiol, <5 % 1,4-Cyclohexandiol, <5 % Monoalkohole und 1-50 Gew.-% oligomere oder polymere Ester der Adipinsäure und 6-Hydroxycarbonsäure (Hochsieder gegenüber 1,6-Hexandiol). Die Zusammensetzung hängt insbesondere ab von der Einstellung des Hydrierumsatzes (erfindungsgemäß einzustellen zwischen 40 und 97 %, bevorzugt zwischen 50 und 95 %). Ein vergleichsweise hoher Hydrierumsatz ist dann einzustellen, wenn bei dem erfindungsgemäßen Verfahren möglichst große Mengen von 1,6-Hexandiol gewonnen werden sollen. Ein vergleichsweise geringer Hydrierumsatz ist dann einzustellen, wenn bei dem erfindungsgemäßen Verfahren möglichst große Mengen an reinem ε-caprolacton hergestellt werden sollen. Bei einer vollständigen Hydrierung würde sämtlicher 6-Hydroxycapronsäureester hydriert werden und somit nicht mehr als Ausgangsmaterial für die Cyclisierung zum ε-Caprolacton zur Verfügung stehen. Auf der anderen Seite ist der Hydrierungsschritt und die anschließende destillative Abtrennung des dabei entstehenden 1,6-Hexandiols nötig, da auf diese Weise 1,2-Cyclohexandiol vor der Cyclisierungsreaktion effizient mit entfernt wird und damit sehr reines und praktisch 1,2-Cyclohexandiol-freies ε-Caprolacton gewonnen werden kann (weniger als 0,05 Gew.-% 1,2-Cyclohexandiol im verbleibenden Esterstrom).

In einer Ausführungsform des erfindungsgemäßen Verfahrens wird ein Teil des Hydrieraustrags, der einen hohen Anteil von 1,6-Hexandiol enthält, direkt oder nach destillativer Reinigung, anstelle von reinem 1,6-Hexandiol für die Veresterung der DCL verwendet. Der Vorteil dieses Verfahrens besteht darin, dass andere Diole, wie 1,5-Pentandiol, 1,4-Butandiol, 1,4-Cyclohexandiol, 1,2-Cyclohexandiol, die zum Teil Nebenprodukte darstellen, das 1,6-Hexandiol ersetzen. Dadurch werden Verluste an 1,6-Hexandiol vermindert und der aufzuarbeitende Hydrierstrom verkleinert sich.

Von dem Hydrieraustrag von Schritt (b) werden im Destillationsschritt (c) des erfindungsgemäßen Verfahrens in einer ersten Kolonne Diole wie 1,6-Hexandiol, 1,5-Pentandiol, 1,4-Butandiol, 1,4-Cyclohexandiol, 1,2-Cyclohexandiol und verschiedene Leichtsieder abgetrennt. Die Kolonne besitzt 1 bis 30 theoretische Böden. Gearbeitet wird bei Sumpftemperaturen von 120 bis 250°C und Drücken von 5 bis 500 mbar.

Das Kopfprodukt aus diesem Destillationsschritt (c) enthält im Allgemeinen 75 bis 95 Gew.-% 1,6-Hexandiol, 3 bis 10 Gew.-% 1,5-Pentandiol, 1,4-Butandiol, 1,2-Cyclohexandiol und 1,4-Cyclohexandiol (jeweils weniger als 5 Gew.-%), weiterhin bis 5 Gew.-% Monoalkohole wie z.B. n-Butanol, n-Pentanol und n-Hexanol und weniger als 5 Gew.-% höher siedende Komponenten gegenüber 1,6-Hexandiol. Für die erfolgreiche Reinigung von Caprolacton in hohen Ausbeuten ist es wichtig, dass die 1,2-Cyclohexandiole im Destillationsschritt über das Kopfprodukt abgetrennt werden.

In einer Ausführungsform des erfindungsgemäßen Verfahrens wird ein Teil des Kopfprodukts vom Destillationsschritt (c) in die Veresterung (Schritt (a)) zurückgeführt. Diese Variante besitzt den Vorteil, dass durch die hohe Reinheit des 1,6-Hexandiol-Stroms die Produktströme im Verfahren verkleinert werden.

In einer Ausführungsform des erfindungsgemäßen Verfahrens wird das gewonnene Destillat einer zweiten Kolonne zugeleitet, in der die Feinreinigung des 1,6-Hexandiols erfolgt. Dabei werden 1,6-Hexandiol Reinheiten von > 97 % erzielt.

Der Sumpfaustrag des Destillationsschritts (c) enthält die in der Hydrierung (Schritt (b)) nicht umgesetzten Ester, insbesondere die Ester der 6-Hydroxycapronsäure und der Adipinsäure mit dem verwendeten Alkohol. Diese Ester sind Hochsieder gegenüber 1,6-Hexandiol.

Der Sumpfaustrag des Destillationsschritts (c) kann ggf. nochmals destilliert werden, um Reste an Hexandiol zu gewinnen (Hochsiedereinengung). Dabei wird unter analogen Bedingungen wie zuvor nochmals ein 1,6-Hexandiol-Kopfprodukt erhalten (>95 % 1,6-Hexandiol), das mit dem vorigen Kopfprodukt vereinigt werden kann.

Der Sumpfaustrag lässt sich durch Methanolyse (Umesterung aller Ester mit Methanol und Analyse der entstandenen Methylester) genauer analysieren und setzt sich demnach (bei Verwendung von 1,6-Hexandiol zur DCL-Veresterung) folgendermaßen zusammen:

10-40 Gew.-% 1,6-Hexandiol (frei vorliegend oder in veresterter Form), 10-40 Gew.-% 6-Hydroxycapronsäure-Äquivalente (6-Hydroxycapronsäure ist im Sumpfstrom als 6-Hydroxycapronsäure-Esterz.B. mit 1,6-Hexandiol enthalten), 1-30 Gew.-% Adipinsäure-Äquivalente (Adipinsäure im Sumpfstrom als Adipinsäureester z.B. mit 1,6-Hexandiol enthalten), 0,1-3 Gew.-% 1,4-Cyclohexandiol, 0,1-10 Gew.-% Bis-(6-hydroxyhexyl)ether (frei vorliegend oder in veresterter Form), dazu sonstige nicht weiter identifizierte Schwersieder. 1,2- Cyclohexandiole können in diesem Strom nicht nachgewiesen werden.

Wichtig für eine hohe Reinheit an ε-Caprolacton ist die Abtrennung der 1,2-Cyclohexandiole vom Hydroxycapronsäureester, da diese Komponenten Azeotrope miteinander bilden. Es war nicht vorauszusehen, dass sich - nach der erfindungsgemäßen Kombination aus Veresterung von DCL mit hoch siedenden Alkoholen (verglichen mit ε-Caprolacton) und katalytischer Hydrierung der entstandenen Ester - die in der DCL enthaltenen 1,2-Cyclohexandiole destillativ zusammen mit dem 1,6-Hexandiol praktisch vollständig von der die 6-Hydroxycapronsäureester enthaltenden Sumpffraktion abtrennen lassen. Dies ermöglicht in wirtschaftlicher Weise die Herstellung von ε-Caprolacton mit einer Reinheit von 99 %, bevorzugt von 99,9 % ausgehend von DCL durch Cyclisierung des praktisch 1,2-Cyclohexandiol-freien (weniger als 0,05 Gew.-%) 6-Hydroxycapronsäureesters.

Die Sumpffraktion aus Schritt (c) wird für die Cyclisierung (Schritt (d)) des 6-Hydroxycapronsäureesters zum ε-Caprolacton verwendet.

Die Cyclisierung (Schritt (d)) erfolgt mit oder ohne Katalysator. Als Katalysator können generell Säuren, insbesondere Lewis-Säuren verwendet werden. Beispiele für solche Katalysatoren sind Titan-, Bor-, Aluminium-, Vanadium-, Eisen-, Zink- oder Zirkonium-Verbindungen. Bevorzugt sind Titan-tetraalkoholate $Ti(OR)_4$, wobei R für aliphatische oder aromatische Reste mit 1-12 Kohlenstoffatomen steht. Die Katalysatoren können eingesetzt werden in Mengen zwischen 0,01 und 1,0 Gew.-%).

Für die Cyclisierung in der Flüssigphase (Schritt (d)) des erfindungsgemäßen Verfahrens wird eine Apparatur mit Kolonne (>1 bis 30 theoretische Trennstufen) verwendet, die kontinuierlich oder diskontinuierlich betrieben werden kann. Gearbeitet wird bei Sumpftemperaturen von 180 und 300°C und bei Drücken zwischen 5 und 500 mbar.

Das Destillat enthält im Allgemeinen (bei Verwendung von Hexandiol als Veresterungsalkohol) 40-75 % Hexandiol, 15-50 % Caprolacton, sowie 1,4-Butandiol, 1,5-Pentandiol, 1,4-Cyclohexandiol, Valerolacton (jeweils <5 %) aber keine 1,2-Cyclohexandiole. Im Sumpf verbleiben üblicherweise insbesondere Adipinsäureester mit Hexandiol und Bis-(6-hydroxyhexyl)ether und sonstige Schwersieder sowie der eingesetzte Cyclisierungskatalysator. Der Sumpf kann je nach Gehalt an verwertbaren Adipinsäureestern zurückgeführt oder verbrannt werden.

Der Sumpfaustrag des Cyclisierungsschritts (d) lässt sich durch Methanolyse genauer analysieren und setzt sich demnach (bei Verwendung von Hexandiol als Veresterungsalkohol) folgendermaßen zusammen: 0-30 Gew.-% 1,6-Hexandiol (frei vorliegend oder in veresterter Form), 0-20 Gew.-% 6-Hydroxycapronsäure-Äquivalente (6-Hydroxycapronsäure ist im Sumpfstrom als 6-Hydroxycapronsäure-Esterz.B. mit 1,6-Hexandiol enthalten), 1-60Gew.-% Adipinsäure-Äquivalente (Adipinsäure im Sumpfstrom als Adipinsäureester z.B. mit Hexandiol enthalten), 0-1 Gew.-% 1,4- Cyclohexandiol, 0,1-30 Gew.-% Bis-(6-hydroxyhexyl)ether (frei vorliegend oder in veresterter Form), dazu sonstige nicht weiter identifizierte Schwersieder.

Die destillative Aufreinigung von ε-Caprolacton (Schritt (e)) kann erfindungsgemäß in einer oder zwei Destillationskolonnen erfolgen. Sie kann diskontinuierlich im Batchverfahren oder kontinuierlich mit Entnahme des Produkts über einen Seitenabzug erfolgen. Bei der Verwendung von zwei Destillationskolonnen erfolgt im Allgemeinen die Abtrennung der Leichtsieder in erster und die Abtrennung der Schwersieder in zweiter Kolonne. Die Destillation erfolgt üblicherweise in Kolonnen mit 1-30 theoretischen Trennstufen. Gearbeitet wird in der Regel bei Sumpftemperaturen von 100-250 °C und bei Drücken zwischen 5 und 500 mbar. Nach Abtrennung der Leichtsieder (1,4-Butandiol, 1,5-Pentandiol, Valerolacton) 1,2-Cyclohexandiol-freies (weniger als 0,1 Gew.-%) ε-Caprolacton in guter Reinheit erhalten. Das Verfahren erlaubt die Herstellung von Caprolacton in einer Reinheit von über 99,9 %. In der Sumpffraktion verbleiben Hexandiol, 1,4-Cyclohexandiol und andere Schwersieder. Bei Bedarf kann aus dem Sumpf noch 1,6-Hexandiol gewonnen werden.

Die vorliegende Erfindung betrifft Verfahren zur Herstellung von 1,6-Hexandiol und ε-Caprolacton aus einer Dicarbonsäurelösung (DCL) umfassend die Schritte

> a) Veresterung von DCL mit Alkoholen,
> b) partielle katalytische Hydrierung des aus Schritt (a) erhaltenen Estergemisches,
> c) Destillation des aus Schritt (b) erhaltenen Hydrieraustrags und dabei Abtrennung des 1,6-Hexandiol enthaltenen Kopfprodukts,
> d) Cyclisierung des 6-Hydroxycapronsäureesters aus der Sumpffraktion von Schritt (c) in Gegenwart mindestens eines Alkohols mit einem Siedepunkt im verwendeten Druckbereich größer dem des ε-Caprolactons, wobei dieser Alkohol frei oder bevorzugt auch als Bestandteil der Ester der Sumpffraktion gebunden vorliegt, und
> (e) Reinigung des ε-Caprolactons aus dem Destillat von Schritt (d) mittels Destillation, wobei die Cyclisierung in Schritt (d) in der Flüssigphase in einer Apparatur mit Kolonne mit mehr als einer theoretischen Trennstufe durchgeführt wird.

Die Alkohole für die Veresterung (Schritt (a)) des erfindungsgemäßen Verfahrens sind einzelne Alkohole mit 1 bis 30 C-Atomen, bevorzugt sind solche, die im Druckbereich von 10 bis 1500 mbar höher sieden als ε-Caprolacton (beispielsweise 96-97°C bei 20 mbar oder 235°C bei 1013 mbar), sowie Gemische davon oder Zusammensetzungen, die solche Alkohole enthalten (bevorzugt zu einem Anteil von mindestens 30 Gew.-%, besonders bevorzugt zu einem Anteil von mindestens 50 Gew.-%). Solche alkoholhaltigen Zusammensetzungen sind beispielsweise der Hydrieraustrag von Schritt (b) oder die Diol-Gemische, die bei der anschließenden Destillation (Schritt (c)) als Kopfprodukt abgetrennt werden können. Dabei können Monoalkohole sowie Diole und andere mehrwertige Alkohole verwendet werden. Besonders geeignet sind mehrwertige Alkohole, insbesondere Diole, insbesondere α,ω-Diole mit sechs bis zwölf Kohlenstoffatomen. Besonders bevorzugt ist 1,6-Hexandiol, da dieser Veresterungsalkohol einem Zielprodukt des erfindungsgemäßen Verfahrens entspricht. Beispiele für derartige Alkohole sind Glycerin, Trimethylolpropan, 1,6-Hexandiol, 1,8-Octandiol, 1,10-Decandiol, 1,12-Dodecandiol, 1-Tridecanol, 1-Pentadecanol, 1-Hexadecanol. 1-Octadecanol, 1-Eicosanol, Butylethylpropandiol, Neopentylglycol, Pentaerythrit, Triethylenglycol, Tetraethylenglycol, Bis(6-hydroxyhexyl)ether.

Insbesondere betrifft die vorliegende Erfindung ein Verfahren zur Herstellung 1,6-Hexandiol und sehr reinem ε-Caprolacton aus einer Dicarbonsäurelösung (DCL) umfassend die Schritte

> (a) Veresterung der DCL mit Alkoholen,
> (b) partielle katalytische Hydrierung des aus Schritt (a) erhaltenen Estergemisches,
> (c) Destillation des aus Schritt (b) erhaltenen Hydrieraustrags und dabei Abtrennung des 1,6-Hexandiol enthaltenen Kopfprodukts,
> (d) Cyclisierung des 6-Hydroxycapronsäureesters aus der Sumpffraktion von Schritt (c) in Gegenwart mindestens eines Alkohols mit einem Siedepunkt im verwendeten Druckbereich größer dem des ε-Caprolactons, wobei dieser Alkohol frei oder auch als Bestandteil der Ester der Sumpffraktion vorliegt, und wobei Alkohole mit einem Siedepunkt größer dem des ε-Caprolactons bereits mit dem Veresterungsschritt (a) als Veresterungsalkohole in das Verfahren eingebracht werden, und
> (e) Reinigung des ε-Caprolactons aus dem Destillat von Schritt (d) mittels Destillation,
> wobei die Cyclisierung in Schritt (d) in der Flüssigphase in einer Apparatur mit Kolonne mit mehr als einer theoretischen Trennstufe durchgeführt wird.

Die Alkohole für die Veresterung (Schritt (a)) des erfindungsgemäßen Verfahrens sind in diesem Fall Alkohole, die im Druckbereich von 10 bis 1500 mbar höher sieden als ε-Caprolacton (beispielsweise 96-97°C bei 20 mbar oder 235°C bei 1013 mbar), sowie Gemische davon oder Zusammensetzungen, die solche Alkohole enthalten (bevorzugt zu einem Anteil von mindestens 30 Gew.-%, besonders bevorzugt zu einem Anteil von mindestens 50 Gew.-%). Solche alkoholhaltigen Zusammensetzungen sind beispielsweise der Hydrieraustrag von Schritt (b) oder die Diol-Gemische, die bei der anschließenden Destillation (Schritt (c)) als Kopfprodukt abgetrennt werden können. Dabei können Monoalkohole sowie Diole und andere mehrwertige Alkohole verwendet werden. Besonders geeignet sind mehrwertige Alkohole, insbesondere Diole, insbesondere α,ω-Diole mit sechs bis zwölf Kohlenstoffatomen. Besonders bevorzugt ist 1,6-Hexandiol, da dieser Veresterungsalkohol einem Zielprodukt des erfindungsgemäßen Verfahrens entspricht. Beispiele für derartige Alkohole sind Glycerin, Trimethylolpropan, 1,6-Hexandiol, 1,8-Octandiol, 1,10-Decandiol, 1,12-Dodecandiol, 1-Tridecanol, 1-Pentadecanol, 1-Hexadecanol. 1-Octadecanol, 1-Eicosanol, Butylethylpropandiol, Neopentylglycol, Pentaerythrit, Triethylenglycol, Tetraethylenglycol, Bis(6-hydroxyhexyl)ether.

Insbesondere betrifft die vorliegende Erfindung ein Verfahren zur Herstellung 1,6-Hexandiol und sehr reinem ε-Caprolacton aus einer Dicarbonsäurelösung (DCL) umfassend die Schritte

(a) Veresterung der DCL mit leicht siedenden Alkoholen,
(b) partielle katalytische Hydrierung des aus Schritt (a) erhaltenen Estergemisches,
(c) Destillation des aus Schritt (b) erhaltenen Hydrieraustrags und dabei Abtrennung des 1,6-Hexandiol enthaltenen Kopfprodukts,
(d) Cyclisierung des 6-Hydroxycapronsäureesters aus der Sumpffraktion von Schritt (c) in Gegenwart mindestens eines Alkohols mit einem Siedepunkt im verwendeten Druckbereich größer dem des ε-Caprolactons, wobei dieser Alkohol im Wesentlichen bei der Hydrierung in Schritt (b) entstandenes 1,6-Hexandiol ist und frei oder auch als Bestandteil der Ester der Sumpffraktion gebunden vorliegt, und
(e) Reinigung des ε-Caprolactons aus dem Destillat von Schritt (d) mittels Destillation,
wobei die Cyclisierung in Schritt (d) in der Flüssigphase in einer Apparatur mit Kolonne mit mehr als einer theoretischen Trennstufe durchgeführt wird, und
wobei für die Hydrierung in Schritt (b) und/oder die Destillation in Schritt (c) unter Bedingungen durchgeführt werden, die eine Verdrängung der leichter siedenden Veresterungsalkohole aus Schritt (a) durch das bei der Hydrierung entstehende 1,6-Hexandiol im Sinne einer Umesterung ermöglichen. Solche Bedingungen sind beispielsweise die Verwendung von Hydrierkatalysator mit sauren oder basischen Zentren für die Hydrierung in Schritt (b), die Gegenwart von in geringe Mengen von Säuren oder Basen (Säure- bzw. Basenzahl im Zulauf zur Destillationsstufe (c) von mindestens 0,01, bevorzugt 0,01 bis 5) bei der Destillation von Schritt (c), und/oder die Gegenwart von Umesterungskatalysatoren bei der Hydrierung von Schritt (b) und/oder der Destillation von Schritt (c).

Geeignete Umesterungskatalysatoren sind beispielsweise Natriummethylat oder Titanate wie Tetra-n-butyltitant, in Mengen bezogen auf den Zulauf von 1 bis 1000 ppm, bevorzugt 5 bis 500 ppm, besonders bevorzugt 10 bis 300 ppm bei dem Hydrierungsschritt (b) bzw. von 1 bis 5000 ppm, bevorzugt 5 bis 3000 ppm, besonders bevorzugt 10 bis 2000 ppm bei dem Destillationsschritt (c). Die Alkohole für die Veresterung (Schritt (a)) des erfindungsgemäßen Verfahrens sind in diesem Fall einzelne Alkohole mit 1 bis 30 C-Atomen, bevorzugt sind solche, die im Druckbereich von 10 bis 1500 mbar leichter sieden als ε-Caprolacton (beispielsweise 96-97°C bei 20 mbar oder 235°C bei 1013 mbar), sowie Gemische davon oder Zusammensetzungen, die solche Alkohole enthalten (bevorzugt zu einem Anteil von mindestens 30 Gew.-%, besonders bevorzugt zu einem Anteil von mindestens 50 Gew.-%). Besonders bevorzugt ist Methanol als Veresterungsalkohol.

Die partielle katalytische Hydrierung der Ester (Schritt (b) des erfindungsgemäßen Verfahrens) ist so eingestellt, dass bevorzugt ein Hydrierumsatz im Bereich von 40 bis 97 %, bevorzugt von 50 bis 95 % erzielt wird.

Die Cyclisierung (Schritt (d)) des erfindungsgemäßen Verfahrens erfolgt in der Flüssigphase in einer Apparatur mit Kolonne mit mehr als einer theoretischen Trennstufe.

Die DCL des erfindungsgemäßen Verfahrens ist erhältlich durch

I) Oxidation von Cyclohexan mit Sauerstoff oder Sauerstoff enthaltenden Gasen zu Gemischen aus Cyclohexanol, Cyclohexanon und Carbonsäuren mit bis zu sechs Kohlenstoffatomen, und
II) Umsatz des nach Schritt (I) erhaltenen Reaktionsgemisches mit Wasser und Abtrennung der DCL aus dem flüssigen zweiphasigen Reaktionsgemisch.

Ein bevorzugter Katalysator für den Hydrierungsschritt (b) des erfindungsgemäßen Verfahrens ist erhältlich durch ein Herstellungsverfahren umfassend die Schritte

(i) Bereitstellung eines oxidischen Material enthaltend Kupferoxid, Aluminiumoxid und mindestens eins der Oxide des Lanthans, Wolframs, Molybdäns, Titans, Zirkoniums oder Eisen,
(ii) Zugabe von pulverförmigem metallischem Kupfer, Kupferblättchen, pulverförmigem Zement, Graphit oder einem Gemisch zu dem oxidischen Material in aus Schritt (i) und
(iii) Formung des aus Schritt (ii) resultierenden Gemisches zu einem Formkörper.

Es war nicht vorauszusehen, dass solche Katalysatoren bei Verwendung von Oligoestern als Feed und Restsäurezahlen von 1 bis 20 über lange Reaktionszeiten eine hohe Aktivität bei hoher Seitendruckfestigkeit und damit hoher mechanischer Festigkeit beibehalten würde

In einer Variante des erfindungsgemäßen Verfahrens wird die eingesetzte DCL zunächst so hydriert, dass darin enthaltene 5-Formylvaleriansäure und gegebenenfalls 6-Hydroperoxycapronsäure in 6-Hydroxycapronsäure und 1,4-Cyclohexandion und 4-Hydroxycyclohexanon in 1,4-Cyclohexandiole umgewandelt werden.

In einer Variante des erfindungsgemäßen Verfahrens wird das 1,6-Hexandiol aus der Kopffraktion der Destillation von Schritt (c) destillativ weiter gereinigt.

Gemäß dem erfindungsgemäßen Verfahren wird das ε-Caprolacton aus dem Destillat von Schritt (d) destillativ weiter gereinigt (Schritt (e)).

Die erfindungsgemäßen Verfahren erlauben in wirtschaftlicher Weise, aus DCL, einem Abfallprodukt der Cyclohexan-Oxidation, 1,6-Hexandiol und sehr reines ε-Caprolacton (Reinheit von mindestens 99 %, bevorzugt von mindestens 99,9%) herzustellen.

Da bei erfindungsgemäßen Verfahren das Sumpfprodukt eines jeden Verfahrensschritts als Ausgangsmaterial für den Folgeschritt verwendet wird, werden Verluste an oder aufwendige Rückführungen von Wertstoffen vermieden.

Das erfindungsgemäße Verfahren erlaubt darüber hinaus in gewissem Maße durch Änderung des Hydrierumsatzes die Modulation des Produktverhältnisses zwischen ε-Caprolacton und 1,6-Hexandiol.

Beschreibung der Abbildung

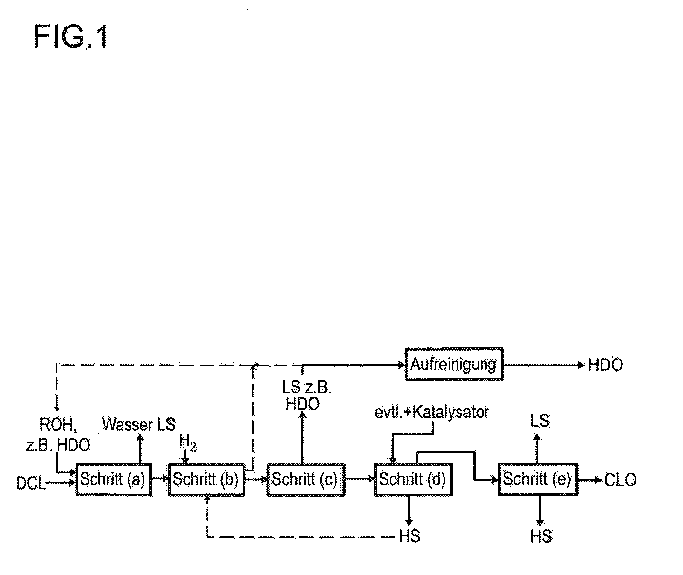

Fig. 1 veranschaulicht das erfindungsgemäße Verfahren. Die eingesetzte Dicarbonsäurelösung (DCL) wird zusammen mit bevorzugt einem hoch siedenden Alkohol (ROH), z.B. 1,6-Hexandiol (HDO) und unter Abtrennung von Leichtsiedern (LS) und Wasser verestert ((Schritt (a)) und anschließend unter Zugabe von Wasserstoff (H$_2$) katalytisch partiell hydriert (Schritt (b)). Von dem Hydrieraustrag werden dann 1,6-Hexandiol (HDO), Leichtsieder und die bei der späteren Reinigung von ε-Caprolacton besonders störende 1,2-Cyclohexandiole abgetrennt (Schritt (c)). Das so abgetrennte 1,6-Hexandiol kann weiter aufgereinigt werden. Die Sumpffraktion der Destillation (Schritt (c)) wird in der anschließenden Cyclisierung (Schritt (d)), gegebenenfalls nach Zugabe eines Cyclisierungskatalysators weiterverarbeitet. Aus der Kopffraktion wird ε-Caprolacton (CLO) gewonnen, das destillativ (Schritt (e)) unter Abtrennung der Leichtsieder (LS) und Schwersieder (HS) weiter zu sehr reinem ε-Caprolacton (CLO) aufgereinigt werden kann. Die Schwersiederfraktion (HS) von Schritt (d) kann gegebenenfalls in den Hydrierungsschritt (b) rückgeführt werden. Teile der alkoholhaltigen Fraktion aus Schritt (b) und/oder (c) können, gegebenenfalls nach weiterer Aufreinigung, für die Veresterung der DCL in Schritt (a) verwendet werden.

Ausführungsbeispiele

Beispiel 1:

1. Abmischung eines Dicarbonsäure-Hexandiolgemischs

Die verwendete Dicarbonsäurelösung (DCL) wurde durch wässrige Extraktion eines Reaktionsaustrags, der aus der Oxidation von Cyclohexan mit Luft stammte, erhalten.

Das für die Veresterung verwendete 1,6-Hexandiol-haltige Diol-Gemisch wurde durch destillative Abtrennung von Hoch- und Leichtsiedern (gegenüber den Diolen) aus dem Hydrieraustrag der Oligo- und Polyester-Hydrierung hergestellt (siehe Stufe 4a und 4b).

Zu 209 kg einer DCL (Säurezahl: 268 mg KOH/g), enthaltend u. a. Adipinsäure (ADS, 21,6 Gew.-

%), 6-Hydroxycapronsäure (HCS, 14,5 Gew.-%) und Wasser (48 Gew.-%), anteilig in Form von Oligomeren (Oligo-Hydroxycapronsäure, Oligomere bestehend aus Adipinsäure- und Hydroxycapronsäure-Einheiten), wurden 94 kg eines 1,6- Hexandiol-haltige Diol-Gemischs zugegeben. Das Hexandiol-haltige Diol-Gemisch enthielt u.a. 1,6-Hexandiol (ca. 83 Gew.-%), 1,5-Pentandiol (ca. 8 Gew.-%), sowie 1,4-Butandiol, 1,4-Cyclohexandiol und 1,2-Cyclohexandiol (jeweils <2 Gew.-%).

2. Herstellung eines oligomeren Estergemisches (Verfahrensschritt (a))

Das DCL-Hexandiol-Gemisch der Stufe 1 wurde kontinuierlich in einen Verdampfer (Entwässerungsstufe, 150°C, Umgebungsdruck) bei einem Durchsatz von 275 g/h dosiert. Dabei wurden Wasser und leicht siedende Komponenten abdestilliert (127 g/h). Der Sumpfaustrag wurde anschließend in eine 5-stufige Rührkesselkaskade überführt (180 g/h, 220°C, 1-1,4 bar abs.), in der die Veresterung bis zum fast vollständigen Umsatz gebracht wurde (Säurezahl: 6 mg KOH/g, entsprechend 98 % Umsatz). In der Esterkaskade wurden ebenfalls leicht siedende Komponenten abdestilliert (14 g/h), die in die Entwässerungsstufe rückgeführt wurden. Man erhielt als Sumpfaustrag ein oligomeres Gemisch, enthaltend hauptsächlich Ester aus den ursprünglich zugeführten Carbonsäurederivaten und Diolen (156 g/h, 57 % Gewichtsausbeute, bezogen auf den gesamten Zulauf, Esterzahl: 348 mg KOH/g).

3. Hydrierung des oligomeren Estergemisches (Verfahrensschritt (b))

Die oligomeren Ester der Stufe 2 wurden mit 60 ppm Natriummethylat versetzt und anschließend kontinuierlich an einem Cu-Katalysator hydriert. Der Katalysator wurde nach WO 2007/6719 Beispiel 3 hergestellt und aktiviert.

Das Reaktorsystem bestand aus einem Hauptreaktor (Rohrreaktor, 400 mL, 600 g Katalysator) und einem Nachreaktor (Rohrreaktor, 100 mL, 150 g Katalysator). Der Hydrierzulauf wurde in Rieselfahrweise über den fest angeordneten Katalysator geleitet. Um die sich bei der Hydrierung entwickelnde Wärme abzuführen, wurde der Hauptreaktor mit Flüssigkeitskreislauf, der Nachreaktor im geraden Durchgang betrieben.

Der Hydrierreaktor wurde 600 h bei 240°C und 255 bar H$_2$ betrieben. Bei einem Zulauf von 250 g/h (Katalysatorbelastung: 0,63 kgL$^{-1}$h$^{-1}$, Hauptreaktor) wurde ein Umsatz von 93 % erzielt (Esterzahl: 24 mg KOH/g). Der Hydrieraustrag wurde anschließend in einem Behälter auf Umgebungsdruck entspannt und auf Umgebungstemperatur gekühlt. Man erhielt Austräge, deren Gehalt an 1,6-Hexandiol 71 Gew.-% betrug. Die Hydrierung verlief mit 92 % Ausbeute zu 1,6-Hexandiol (die Ausbeute bezieht sich auf die in der DCL vorhandenen C$_6$-Komponenten, die durch Hydrierung zu 1,6-HDO führen können:

6-Hydroxycapronsäure, 6-Oxocapronsäure (5-Formylvaleriansäure), Adipinsäure und Dihydromuconsäure).

Der Gehalt an 1,6-Hexandiol wurde per Gaschromatographie ermittelt: DB-5 (Agilent J&W), 30 m x 0,2 mm x 1 $\mu$m; Temperaturprofil: 60°C (5 min) auf 220°C (16°C/min, 10 min) auf 260°C (20°C/min, 21 min) auf 290°C (20°C/min, 10 min). Diethylenglykol Dimethylether (DEGDME) wurde als interner Standard verwendet, tR (DEGDME) = 8,8 min, 40 tR (1.6-Hexandiol) = 11,8 min.

4a. Abtrennung von Hochsiedern aus dem Hydrieraustrag (Verfahrensschritt (c))

In einer Destillationsblase mit aufgesetzter Kolonne (DN50, Einbauten 1 m, Gewebepackung 750 $m^2/m^3$) wurden Hydrieraustäge (38 kg) der Stufe 3 destillativ aufgetrennt. Bei 50 mbar und 178°C Sumpftemperatur wurden 32 kg Kopfprodukt erhalten (Rücklaufverhältnis 1:1), das 81 Gew.-% 1,6-Hexandiol enthielt (daneben 8 Gew.-% Pentandiol, 2 Gew.-% 1,2-Cyclohexandiol, 1,4 Gew.-% 1,4-Cyclohexandiol, sonstiges 7 Gew.-%). Weiterhin fielen 6 kg Sumpfprodukt an, das laut Gaschromatographie 33 Gew.-% 1,6-Hexandiol enthält, sowie Adipinsäure- und Hydroxycapronsäureester (nicht quantifiziert).

4b. Hochsiedereinengung

In einer Destillationsblase mit aufgesetzter Kolonne (DN50, Einbauten 1 m, Gewebepackung 750 $m^2/m^3$) wurden die Sumpfausträge (1,7 kg) der Stufe 4a destillativ eingeengt. Dabei wurde bei 20 mbar und einer Sumpftemperatur von 220°C (Rücklaufverhältnis 17:1) 0,54 kg Kopfprodukt erhalten, das einen Hexandiol-Anteil > 95% aufwies. Als Sumpfprodukt wurden 1,16 kg eingeengte Schwersieder erhalten. Laut Gaschromatographie enthielt dieser Strom noch 9% Hexandiol.

Durch Methanolyse dieses Stroms wurde die Zusammensetzung genauer bestimmt: dazu wurden 15 g des Sumpfaustrags mit 150 ml Methanol und 0,05 g Tetra-n-butyltitanat versetzt und in einem 300 ml Autoklaven 6 Stunden auf 170 °C erhitzt. Der Gehalt an 1,6-Hexandiol (HDO), 6-Hydroxycapronsäuremethylester (HCSMe), Adipinsäuredimethylester (ADSDMe), 1,2-Cyclohexandiol (1,2-CHDO) und 1,4-Cyclohexandiol (1,4-CHDO) wurde per Gaschromatographie bestimmt (DB-5, 30 m x 0,23 mm x 1 $\mu$m, 60 °C (5 min) auf 220°C (16 °C/min, 10 min) auf 260°C (20°C/min, 10 min); Diethylengylkoldimethylether als interner Standard, Retentionszeit 1,2-CHDO: 10,8 min; 1,4-CHDO: 11,1 und 11,2 min, HDO: 11,5 min; HCSMe: 12,1 min; ADSDMe: 12,6 min; DiHDO: 19,2 min). Demnach enthielt die eingesetzte Probe 22,7 Gew.-% HDO, 14,2 Gew.-% 6-Hydroxycapronsäure (HCS), 8,9 Gew.-% Adipinsäure (ADS), 0,6 Gew.-% 1,4-CHDO und 7,2 Gew.-% DiHDO. 1,2-CHDO war in dieser Fraktion nicht nachzuweisen.

5. Cyclisierung (Verfahrensschritt (d))

Der in Stufe 4b angefallene Sumpfstrom (501 g) wurde in das Sumpfgefäß einer Destillationskolonne gegeben (30 cm, befüllt mit 5x5 mm Glasraschigringen) und mit 1 g Tetra-n-butyltitanat versetzt. Bei 30 mbar wurde eine Sumpftemperatur von anfangs 180°C eingestellt, die mit zunehmender Destillationsdauer kontinuierlich bis auf 270°C erhöht wurde (Rücklaufverhältnis 50:10). Insgesamt wurden 198 g Kopfprodukt erhalten, das folgende Komponenten enthielt: 4 Gew.-% Pentandiol, 1 Gew.-% $\delta$-Valerolacton, 1 Gew.-% 1,4-Cyclohexandiol, 55 Gew.-% 1,6-Hexandiol, 30 Gew.-% $\epsilon$-Caprolacton). Dies entspricht einer $\epsilon$-Caprolacton-Ausbeute von 97 % bezogen auf die im eingesetzten Strom enthaltenen Hydroxycapronsäure-Einheiten und einer 1,6-Hexandiol-Ausbeute von 96 % bezogen auf die eingesetzten Hexandiol-Einheiten. Es waren im Destillat keine 1,2-Cyclohexandiole nachweisbar.

Als Sumpfaustrag wurden 271 g erhalten. Laut Methanolyse (analog zu Stufe 4b) enthielt dieser Austrag noch 0,5 Gew.-% 1,6-Hexandiol, 0,4 Gew.-% Hydroxycapronsäure, 13,9 Gew.-% Adipinsäure und 12,5 Gew.-% Bis-(6-hydroxyhexyl)ether. 1,2- und 1,4-Cyclohexandiole waren in dieser Fraktion nicht nachweisbar.

6. $\epsilon$-Caprolacton-Reindestillation

Das in Stufe 5 angefallene, $\epsilon$-Caprolacton enthaltende Destillat wurde in das Sumpfgefäß einer Destillationskolonne gegeben (1 m, befüllt mit 5x5 Metall-Raschigringen). Die Mischung wurde bei 10 mbar und einer Sumpftemperatur von 145°C durchgeführt (Rücklaufverhältnis 20:10). Nach Abnahme von 30 g Vorlauf ($\epsilon$-Caprolacton-Anteil von 20 Gew.-%) wurde der Hauptlauf bei einer Sumpftemperatur von 150-155°C genommen (41 g, $\epsilon$-Caprolacton-Anteil 99,9 %, Rest Valerolacton). Anschließend wurde die Destillation beendet. Bei Bedarf könnte auch noch das im Sumpf zurückgebliebene Hexandiol abdestilliert werden.

Beispiel 2:

Vergleichsbeispiel analog zu dem in EP 883591 A beschriebenen Verfahren ("Variante E"): Cyclisierung aus einem mit Hexandiol veresterten DCL-Strom.

707 g eines analog zu Beispiel 1, Stufe 2 gewonnenen Estergemisches (laut Methanolyse 0,2 Gew.-% 1,2-Cyclohexandiol, 1,6 Gew.-% 1,4- Cyclohexandiol, 45,4 Gew.-% Hexandiol, 17,7 Gew.-% 6-Hydroxycapronsäure-Äquivalente, 20,0 Gew.-% Adipinsäure-Äquivalente) wurden in das Sumpfgefäß einer 1 m Destillationskolonne (gefüllt mit 5x5 mm Maschendrahtgeweberingen) eingefüllt und ohne Zugabe von Titanat bei 20 mbar und einer Sumpftemperatur zwischen 200 und 246°C destilliert (Rücklaufverhältnis 10:1). Es wurden 65 g eines Destillats erhalten, das folgende Zusammensetzung aufwies: 1,0 Gew.-% 1,2-Cyclohexandiole, 5,4 Gew.-% 1,4-Cyclohexandiole, 22 Gew.-% Hexandiol, 44 Gew.-% Caprolacton. Daneben wurden noch eine Reihe nicht näher identifizierter Nebenprodukte nachgewiesen.

In der ersten Destillationsstufe wurden somit 46 % der eingesetzten 1,2- Cyclohexandiole, 31% des eingesetzten 1,4- Cyclohexandiole, 4 % des Hexandiols und 26 % des Caprolacton abdestilliert.

Zu dem zurückgebliebenen Sumpf wurden 0,5 g Tetra-n-butyltitanat gegeben und erneut bei 20 mbar und einer Sumpftemperatur zwischen 210 und 255°C destilliert (Rücklaufverhältnis 10:1). Dabei wurden insgesamt 154 g eines Destillats erhalten, das die folgende Zusammensetzung aufwies: 0,4 Gew.-% 1,2- Cyclohexandiole, 2,1 Gew.-% 1,4- Cyclohexandiole, 29 Gew.-% Hexandiol, 44 Gew.-% Caprolacton. Daneben wurden noch eine Reihe nicht näher identifizierter Nebenprodukte nachgewiesen.

In der zweiten Destillationsstufe wurden somit 44 % der 1,2- Cyclohexandiol-Menge, 29 % der 1,4- Cyclohexandiol-Menge, 14 % der Hexandiol-Menge und 63 % der Caprolacton-Menge abdestilliert.

Es gelingt hier also nicht, die 1,2- Cyclohexandiole vor der eigentlichen Caprolacton-Gewinnung destillativ so abzutrennen, dass eine hohe Reinheit von Caprolacton erreicht werden kann.

**Patentansprüche**

1. Verfahren zur Herstellung von 1,6-Hexandiol und ε-Caprolacton umfassend folgende Schritte

   a) Veresterung von DCL mit einem Alkohol,
   b) partielle katalytische Hydrierung des aus Schritt (a) erhaltenen Estergemischs,
   c) Destillation des aus Schritt (b) erhaltenen Hydrieraustrags und dabei Abtrennung des 1,6-Hexandiol enthaltenen Kopfprodukts,
   d) Cyclisierung des 6-Hydroxycapronsäureesters aus der Sumpffraktion von Schritt (c) in Gegenwart mindestens eines Alkohols mit einem Siedepunkt im verwendeten Druckbereich größer dem des ε-Caprolactons, einem Gemisch davon oder einer Zusammensetzung, die solche Alkohole enthält, wobei dieser Alkohol frei oder auch als Bestandteil der Ester der Sumpffraktion gebunden ist, und
   e) Reinigung des ε-Caprolactons aus dem Destillat von Schritt (d) mittels Destillation,

   wobei die Cyclisierung in Schritt (d) in der Flüssigphase in einer Apparatur mit Kolonne mit mehr als einer theoretischen Trennstufe durchgeführt wird.

2. Das Verfahren gemäß Anspruch 1, wobei die Cyclisierung des 6-Hydroxycapronsäureesters bei Schritt (d) in Gegenwart mindestens eines Alkohols mit einem Siedepunkt im verwendeten Druckbereich größer dem des ε-Caprolactons, wobei dieser Alkohol als Bestandteil der Ester der Sumpffraktion gebunden ist, durchgeführt wird.

3. Das Verfahren gemäß Anspruch 1 oder 2, wobei die Veresterung in Schritt (a) mit einem Alkohol, der im Druckbereich von 10 bis 1500 mbar höher siedet als ε-Caprolacton, einem Gemisch davon oder einer Zusammensetzung, die solche Alkohole enthält, durchgeführt wird.

4. Das Verfahren gemäß einem der Ansprüche 1 bis 3, wobei die für die Veresterung in Schritt (a) verwendeten Alkohole mehrwertige Alkohole, insbesondere Diole sind.

5. Das Verfahren gemäß Anspruch 3 oder 4, wobei ein Teil des Hydrieraustrags von Schritt (b) als Alkohol enthaltende Zusammensetzung für die Veresterung der DCL in Schritt (a) verwendet wird.

6. Das Verfahren gemäß einem der Ansprüche 3 bis 5, wobei ein Teil der Alkohol enthaltenden Zusammensetzung, die als Kopfprodukt aus der Destillation in Schritt (c) gewonnen wird, für die Veresterung der DCL in Schritt (a) verwendet wird.

7. Das Verfahren gemäß einem der Ansprüche 3 bis 6, wobei 1,6-Hexandiol für die Veresterung der DCL in Schritt (a) verwendet wird.

8. Das Verfahren gemäß Anspruch 1 oder 2, wobei die Veresterung in Schritt (a) mit einem Alkohol, der im Druckbereich von 10 bis 1500 mbar leichter siedet als ε-Caprolacton, einem Gemisch davon oder einer Zusammensetzung, die solche Alkohole enthält, durchgeführt wird, und wobei die Hydrierung in Schritt (b) und/oder die Destillation in Schritt (c) unter Bedingungen durchgeführt werden, die eine Verdrängung des leichter siedenden Veresterungsalkohols aus Schritt (a) durch das bei der Hydrierung entstehende 1,6-Hexandiol im Sinne einer Umesterung ermöglichen.

9. Das Verfahren gemäß Anspruch 8, wobei die Umesterung ermöglichenden Bedingungen die Verwendung eines Hydrierkatalysators mit sauren oder basischen Zentren in Schritt (b), die Gegenwart von Säuren oder Basen in Schritt (c) in einer Menge, die im Zulauf zur Destillationsstufe eine Säure- bzw. Basenzahl von mindestens 0,01 bewirkt, und/oder die Gegenwart von Umesterungskatalysatoren in Schritt (b) und/oder (c) in Mengen bezogen auf den Zulauf von mindestens 1 ppm sind.

10. Das Verfahren gemäß einem der Ansprüche 1 bis 9, wobei die partielle Hydrierung bei Schritt (b) so eingestellt ist, dass ein Hydrierumsatz im Bereich von 40 bis 97 % erzielt wird.

11. Das Verfahren gemäß einem der Ansprüche 1 bis 10, wobei die DCL gewonnen wird durch

   I) Oxidation von Cyclohexan mit Sauerstoff oder Sauerstoff enthaltenden Gasen zu Gemischen aus Cyclohexanol, Cyclohexanon und Carbonsäuren mit bis zu sechs Kohlenstoffatomen, und
   II) Umsatz des nach Schritt (I) erhaltenen Reaktionsgemisches mit Wasser und Abtrennung der DCL aus dem flüssigen zweiphasigen Reaktionsgemisch.

12. Das Verfahren gemäß einem der Ansprüche 1 bis 11, wobei das 1,6-Hexandiol aus der Kopffraktion der Destillation von Schritt (c) destillativ weiter gereinigt wird.

13. Das Verfahren gemäß einem der Ansprüche 1 oder 3 bis 12, wobei die Alkohol enthaltende Zusammensetzung zu einem Anteil von mindestens 30 Gew.-% aus den entsprechenden Alkoholen besteht.

14. Das Verfahren gemäß einem der Ansprüche 1 bis 13, wobei die Hydrierung in Schritt (b) des Veresterungsgemischs aus Schritt (a) in der Flüssigphase in Gegenwart eines Katalysator-Formkörpers hydriert, dessen Vorläufer erhältlich ist durch

   (i) Bereitstellung eines oxidischen Materials enthaltend Kupferoxid, Aluminiumoxid und mindestens eins der Oxide des Lanthans, Wolframs, Molybdäns, Titans, Zirkoniums oder Eisen,
   (ii) Zugabe von pulverförmigem metallischem Kupfer, Kupferblättchen, pulverförmigem Zement, Graphit oder einem Gemisch zu dem oxidischen Material in aus Schritt (i) und
   (iii) Formung des aus Schritt (ii) resultierenden Gemisches zu einem Formkörper.

15. Das Verfahren gemäß einem der Ansprüche 1 bis 14, wobei die Veresterung in Schritt (a) ohne Katalysator-Zusatz durchgeführt wird.

16. Das Verfahren gemäß einem der Ansprüchen 1 bis 15, wobei im Schritt (c) 1,2-Cyclohexandiole über Kopf abgetrennt werden.

**Claims**

1. A process for preparing 1,6-hexanediol and ε-caprolactone, comprising the following steps:

   a) esterification of DCS with an alcohol,

   b) partial catalytic hydrogenation of the ester mixture obtained from step (a),
   c) distillation of the hydrogenation output obtained from step (b) to remove the 1,6-hexanediol-comprising top product,
   d) cyclization of the 6-hydroxycaproic ester from the bottoms fraction from step (c) in the presence of at least one alcohol having a boiling point greater than that of ε-caprolactone within the pressure range used, a mixture of such alcohols or a composition which comprises such alcohols, said alcohol being in free or else bound form as a constituent of the esters of the bottoms fraction, and
   e) purification of the ε-caprolactone from the distillate from step (d) by means of distillation,

   the cyclization in step (d) being performed in the liquid phase in an apparatus with a column having more than one theoretical plate.

2. The process according to claim 1, wherein the cyclization of the 6-hydroxycaproic ester in step (d) is performed in the presence of at least one alcohol having a boiling point greater than that of ε-caprolactone within the pressure range used, said alcohol being in bound form as a constituent of the esters of the bottoms fraction.

3. The process according to claim 1 or 2, wherein the esterification in step (a) is performed with an alcohol having a higher boiling point than ε-caprolactone within the pressure range from 10 to 1500 mbar, a mixture thereof or a composition which comprises such alcohols.

4. The process according to any of claims 1 to 3, where the alcohols used for the esterification in step (a) are polyhydric alcohols, especially diols.

5. The process according to claim 3 or 4, wherein a portion of the hydrogenation output from step (b) is used as the alcohol-comprising composition for the esterification of the DCS in step (a).

6. The process according to any of claims 3 to 5, wherein a portion of the alcohol-comprising composition which is obtained as the top product from the distillation in step (c) is used for the esterification of the DCS in step (a).

7. The process according to any of claims 3 to 6, wherein 1,6-hexanediol is used for the esterification of the DCS in step (a).

8. The process according to claim 1 or 2, wherein the esterification in step (a) is performed with an alcohol having a lower boiling point than ε-caprolactone within the pressure range from 10 to 1500 mbar, a mixture of such alcohols or a composition which comprises such alcohols, and
wherein the hydrogenation in step (b) and/or the distillation in step (c) are performed under conditions which enable displacement of the relatively low-boiling esterification alcohol from step (a) by the 1,6-hexanediol formed in the hydrogenation in the manner of a transesterification.

9. The process according to claim 8, wherein the transesterification-enabling conditions are the use of a hydrogenation catalyst having acidic or basic sites in step (b), the presence of acids or bases in step (c) in an amount which causes an acid or base number of at least 0.01 in the feed to the distillation stage, and/or the presence of transesterification catalysts in step (b) and/or (c) in amounts of at least 1 ppm based on the feed.

10. The process according to any of claims 1 to 9, wherein the partial hydrogenation in step (b) is adjusted such that a hydrogenation conversion in the range from 40 to 97% is achieved.

11. The process according to any of claims 1 to 10, wherein the DCS is obtained by

I) oxidation of cyclohexane with oxygen or oxygen-comprising gases to give mixtures of cyclohexanol, cyclohexanone and carboxylic acids having up to six carbon atoms, and
II) reaction of the reaction mixture obtained after step (I) with water and removal of the DCS from the liquid biphasic reaction mixture.

12. The process according to any of claims 1 to 11, wherein the 1,6-hexanediol from the top fraction of the distillation of step (c) is purified further by distillation.

13. The process according to any of claims 1 or 3 to 12, wherein the alcohol-comprising composition consists of the corresponding alcohols in a proportion of at least 30% by weight.

14. The process according to any of claims 1 to 13, wherein the hydrogenation in step (b) of the esterification mixture from step (a) is hydrogenated in the liquid phase in the presence of a shaped catalyst body, the precursor of which is obtainable by

(i) providing an oxidic material comprising copper oxide, aluminum oxide and at least one of the oxides of lanthanum, of tungsten, of molybdenum, of titanium, of zirconium or of iron,
(ii) adding pulverulent metallic copper, copper flakes, pulverulent cement, graphite or a mixture to the oxidic material from step (i) and
(iii) shaping the mixture resulting from step (ii) to a shaped body.

15. The process according to any of claims 1 to 14, wherein the esterification in step (a) is performed without addition of catalyst.

16. The process according to any of claims 1 to 15, wherein 1,2-cyclohexanediols are removed via the top in step (c).

## Revendications

1. Procédé de fabrication de 1,6-hexanediol et d'ε-caprolactone, comprenant les étapes suivantes :

a) l'estérification de DCL avec un alcool,
b) l'hydrogénation catalytique partielle du mélange d'esters obtenu à l'étape (a),
c) la distillation de la sortie d'hydrogénation obtenue à l'étape (b) et la séparation du produit de tête contenant du 1,6-hexanediol,
d) la cyclisation de l'ester de l'acide 6-hydroxycaproïque à partir de la fraction de fond de l'étape (c) en présence d'au moins un alcool ayant un point d'ébullition dans la plage de pression utilisée supérieur à celui de l'ε-caprolactone, d'un mélange de celui-ci ou d'une composition contenant de tels alcools, cet alcool étant libre ou étant lié en tant que constituant des esters de la fraction de fond, et
e) la purification de l'ε-caprolactone à partir du distillat de l'étape (d) par distillation,

la cyclisation à l'étape (d) étant réalisée en phase liquide dans un appareil comprenant une colonne contenant plus d'une étape de séparation théorique.

2. Procédé selon la revendication 1, dans lequel la cyclisation de l'ester de l'acide 6-hydroxycaproïque à l'étape (d) est réalisée en présence d'au moins un alcool ayant un point d'ébullition dans la plage de pression utilisée supérieur à celui de l'ε-caprolactone, cet alcool étant lié en tant que constituant des esters de la fraction de fond.

3. Procédé selon la revendication 1 ou 2, dans lequel l'estérification à l'étape (a) est réalisée avec un alcool qui présente dans la plage de pression de 10 à 1 500 mbar un point d'ébullition supérieur à celui de l'ε-caprolactone, un mélange de celui-ci ou une composition contenant de tels alcools.

4. Procédé selon l'une quelconque des revendications 1 à 3, dans lequel les alcools utilisés pour l'estérification à l'étape (a) sont des alcools polyvalents, notamment des diols.

5. Procédé selon la revendication 3 ou 4, dans lequel une partie de la sortie d'hydrogénation de l'étape (b) est utilisée en tant que composition contenant un alcool pour l'estérification de DCL à l'étape (a).

6. Procédé selon l'une quelconque des revendications 3 à 5, dans lequel une partie de la composition contenant un alcool qui est obtenue en tant que produit de tête de la distillation à l'étape (c) est utilisée pour l'estérification de DCL à l'étape (a).

7. Procédé selon l'une quelconque des revendications 3 à 6, dans lequel le 1,6-hexanediol est utilisé pour l'estérification de DCL à l'étape (a).

8. Procédé selon la revendication 1 ou 2, dans lequel l'estérification à l'étape (a) est réalisée avec un alcool qui présente dans la plage de pression de 10 à 1 500 mbar un point d'ébullition inférieur à celui de l'ε-caprolactone, un mélange de celui-ci ou une composition contenant de tels alcools, et dans lequel l'hydrogénation à l'étape (b) et/ou la distillation à l'étape (c) sont réalisées dans des conditions qui permettent une élimination de l'alcool d'estérification de point d'ébullition plus faible de l'étape (a) par le 1,6-hexanediol formé lors de l'hydrogénation au sens d'une transestérification.

9. Procédé selon la revendication 8, dans lequel les conditions permettant la transestérification sont :

l'utilisation d'un catalyseur d'hydrogénation comprenant des centres acides ou basiques à l'étape (b),
la présence d'acides ou de bases à l'étape (c) en une quantité qui entraîne un indice d'acidité ou de basicité dans l'alimentation de l'étape de distillation d'au moins 0,01, et/ou
la présence de catalyseurs de transestérification à l'étape (b) et/ou (c) en quantités par rapport à l'alimentation d'au moins 1 ppm.

10. Procédé selon l'une quelconque des revendications 1 à 9, dans lequel l'hydrogénation partielle à l'étape (b) est ajustée de manière à obtenir une conversion d'hydrogénation dans la plage allant de 40 à 97 %.

11. Procédé selon l'une quelconque des revendications 1 à 10, dans lequel la DCL est obtenue par :

I) oxydation de cyclohexane avec de l'oxygène ou des gaz contenant de l'oxygène en mélanges de cyclohexanol, cyclohexanone et acides carboxyliques contenant jusqu'à six atomes de carbone, et
II) mise en réaction du mélange réactionnel obtenu à l'étape (I) avec de l'eau et séparation de la DCL du mélange réactionnel liquide biphasé.

12. Procédé selon l'une quelconque des revendications 1 à 11, dans lequel le 1,6-hexanediol issu de la fraction de tête de la distillation de l'étape (c) est davantage purifié par distillation.

13. Procédé selon l'une quelconque des revendications 1 ou 3 à 12, dans lequel la composition contenant un alcool est constituée des alcools correspondants en une proportion d'au moins 30 % en poids.

14. Procédé selon l'une quelconque des revendications 1 à 13, dans lequel l'hydrogénation à l'étape (b) du mélange d'estérification de l'étape (a) est réalisée en phase liquide en présence d'un corps moulé catalytique, dont le précurseur peut être obtenu par :

(i) préparation d'un matériau oxydique contenant de l'oxyde de cuivre, de l'oxyde d'aluminium et au moins un des oxydes de lanthane, tungstène, molybdène, titane, zirconium ou fer,
(ii) ajout de cuivre métallique en poudre, de feuillets de cuivre, de ciment en poudre, de graphite ou d'un mélange au matériau oxydique de l'étape (i), et
(iii) façonnage du mélange résultant de l'étape (ii) en un corps moulé.

15. Procédé selon l'une quelconque des revendications 1 à 14, dans lequel l'estérification à l'étape (a) est réalisée sans ajout de catalyseur.

16. Procédé selon l'une quelconque des revendications 1 à 15, dans lequel des 1,2-cyclohexanediols sont séparés par la tête à l'étape (c).

# FIG.1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9925672 A1
- DE 1618143
- DE 2013525
- EP 349861 A
- DE 19607954
- WO 9731883 A1
- WO 2008152001 A1
- DE 1951250 A

- EP 847979 A
- US 3524892 A
- EP 0552463 A
- WO 2004085356 A
- WO 2006005505 A
- WO 2007006719 A
- WO 20076719 A

**In der Beschreibung aufgeführte Nicht-Patentliteratur**

- Ullmann's Encyclopedia of Industrial Chemistry. 1987, vol. A8, 219
- **H. KROPF.** Houben-Weyl, Methoden der Organischen Chemie. Georg Thieme Verlag, 1980, 45-67

- **HOUBEN-WEYL.** *Methoden der Organischen Chemie,* vol. IV/Ic, 16-26